(19) Europäisches Patentamt / European Patent Office / Office européen des brevets

(11) **EP 3 377 558 B1**

(12) **EUROPÄISCHE PATENTSCHRIFT**

(45) Veröffentlichungstag und Bekanntmachung des Hinweises auf die Patenterteilung:
**17.06.2020 Patentblatt 2020/25**

(21) Anmeldenummer: **16774653.6**

(22) Anmeldetag: **27.09.2016**

(51) Int Cl.:
*C08G 77/04* (2006.01)      *C08G 77/06* (2006.01)
*C08G 77/08* (2006.01)      *A61K 8/89* (2006.01)
*A61K 8/891* (2006.01)      *C08G 77/18* (2006.01)

(86) Internationale Anmeldenummer:
**PCT/EP2016/072998**

(87) Internationale Veröffentlichungsnummer:
**WO 2018/059670 (05.04.2018 Gazette 2018/14)**

(54) **VERFAHREN ZUR HERSTELLUNG SPHÄRISCHER POLYSILSESQUIOXANPARTIKEL**

PROCESS FOR PRODUCING SPHERICAL POLYSILSESQUIOXANE  PARTICLES

PROCÉDÉ DE PRODUCTION DES PARTICULES DE POLYSILESQUIOXANE SPHÉRIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.09.2018 Patentblatt 2018/39**

(73) Patentinhaber: **Wacker Chemie AG**
**81737 München (DE)**

(72) Erfinder:
• **KNÖR, Sebastian**
**84547 Emmerting (DE)**
• **SEILINGER, Kathrin**
**84489 Burghausen (DE)**

(74) Vertreter: **Fritz, Helmut et al**
**Wacker Chemie AG**
**Zentralbereich Patente, Marken und Lizenzen**
**Hanns-Seidel-Platz 4**
**81737 München (DE)**

(56) Entgegenhaltungen:
• **DATABASE WPI Week 199817 Thomson Scientific, London, GB; AN 1998-189384 XP002763903, -& JP H10 45914 A (TOSHIBA SILICONE KK) 17. Februar 1998 (1998-02-17)**
• **DATABASE WPI Week 200422 Thomson Scientific, London, GB; AN 2004-230738 XP002763904, -& JP 2003 335860 A (TOSHIBA SILICONE KK) 28. November 2003 (2003-11-28)**
• **DATABASE WPI Week 200055 Thomson Scientific, London, GB; AN 2000-581615 XP002763905, -& JP 2000 186148 A (TOSHIBA SILICONE KK) 4. Juli 2000 (2000-07-04) & JP 3 970449 B2 5. September 2007 (2007-09-05) in der Anmeldung erwähnt**

**Beschreibung**

Die Erfindung betrifft ein Verfahren zur Herstellung sphärischer Polysilsesquioxanpartikel durch Hydrolyse von Trialkoxysilan und Kondensation des Hydrolysats.

Der Stand der Technik, beispielsweise JP3970449B2, JPH1045914A, JP2003335860A, JPH06248081A und JPH0488023A kennt verschiedene Verfahren zur Erzeugung von sphärischen Polymethylsilsesquioxanpartikeln. JP3970449B2 beschreibt die Optimierung der Raum-Zeit-Ausbeute und der Kontrolle der Partikelgröße. Bisher ist kein Verfahren beschrieben, welches eine Kontrolle des Agglomerisationsverhaltens der Partikel erlaubt.

Gegenstand der Erfindung ist ein Verfahren zur Herstellung sphärischer Polysilsesquioxanpartikel, bei dem in einem ersten Schritt Trialkoxysilan der allgemeinen Formel (I)

$$RSi(OR^1)_3 \qquad (I),$$

in der

R einen Kohlenwasserstoffrest mit 1 bis 16 Kohlenstoffatomen dessen Kohlenstoffkette durch nicht benachbarte Gruppen -O-unterbrochen sein kann,
$R^1$ einen $C_1$- bis $C_4$-Alkylrest bedeuten,
mit angesäuertem Wasser mit einem pH-Wert von höchstens 6 unter Durchmischung zu einem Hydrolysat umgesetzt wird,
in einem zweiten Schritt das Hydrolysat mit einer Lösung von Alkalihydroxid, das ausgewählt wird aus NaOH und KOH oder einem Gemisch davon vermischt wird
und in einem dritten Schritt die Mischung mindestens 8 h aufbewahrt wird bevor die Polysilsesquioxanpartikel isoliert werden.

Es wurde gefunden, dass durch den Einsatz einer Lösung eines Alkalihydroxids, das ausgewählt wird aus NaOH und KOH in Kombination der vorstehend beschriebenen Verfahrensparameter hochgradig agglomerisationsfreie sphärische Polysilsesquioxanpartikel erhalten werden können. Eine Mahlung der Partikel ist nicht erforderlich. Solche Partikel zeigen ein sehr vorteilhaftes Verhalten, insbesondere für kosmetische Anwendungen. Sie gehen bereits bei geringer Scherung in einen Flüssigkeits-ähnlichen fließfähigen Zustand über (Fluidisieren) und sind dadurch außerordentlich leicht zu verteilen und bewirken ein samtiges Hautgefühl. Dieses Verhalten ist bei agglomerierten Partikeln nicht zu beobachten. Diese ballen beim Verteilen auf der Haut zusammen.

Fluides, also flüssigkeits-ähnliches Verhalten, zeigt sich insbesondere direkt nach dem Aufschütteln der Polysilsesquioxanpartikel. Das fluide Verhalten ist umso stärker ausgeprägt, umso stärker die Volumenzunahme ist. Ein Material, das 50% Volumenzunahme aufweist, zeigt bereits fluides Verhalten, was sich beispielsweise darin äußert, dass das Material im Gebinde - unmittelbar nach dem Aufschütteln - beim Schwenken des Gebindes ähnlich einer Flüssigkeit hin und her fließt. Ein Material mit 50% Volumenzunahme sedimentiert sehr schnell und geht in den nicht-fluiden Ausgangszustand zurück, was nachteilig ist. Die sphärischen Polysilsesquioxanpartikel zeigen vorzugsweise mindestens 100% Volumenzunahme.

Die getrockneten ungemahlenen Polysilsesquioxanpartikel weisen vorzugsweise mindestens 30 Gew.-%, bevorzugt mindestens 40 Gew.-%, besonders bevorzugt mindestens 50 Gew.-%, einer Siebfraktion <20 μm auf.

Die getrockneten ungemahlenen Polysilsesquioxanpartikel weisen vorzugsweise mindestens 60 Gew.-%, bevorzugt mindestens 70 Gew.-%, einer Siebfraktion <40 μm auf.

Die getrockneten ungemahlenen Polysilsesquioxanpartikel weisen vorzugsweise weniger als 25 Gew.-%, bevorzugt weniger als 20 Gew.-%, besonders bevorzugt weniger als 15 Gew.-%, einer Siebfraktion >100 μm auf.

R bedeutet vorzugsweise einen Alkylrest mit 1 bis 6 Kohlenstoffatomen oder Phenylrest, insbesondere Ethylrest oder Methylrest.

$R^1$ bedeutet vorzugsweise einen Methyl-, Ethyl-, oder n-Propylrest, insbesondere einen Methylrest.

Bevorzugte Trialkoxysilane der allgemeinen Formel (I) sind Methyltrimethoxysilan, Methyltriethoxysilan, Methyltri-n-propoxysilan, Methyltriisopropoxysilan und Methyltris(2-methoxyethoxy)silan und Gemische davon.

Die Umsetzung zu einem Hydrolysat erfolgt vorzugsweise in angesäuertem Wasser mit einem pH-Wert von höchstens 5,5 besonders bevorzugt höchstens 4,5 und vorzugsweise mindestens 1, besonders bevorzugt mindestens 2, insbesondere mindestens 2,3.

Das eingesetzte Wasser ist vorzugsweise entsalzt und weist vor dem Ansäuern vorzugsweise eine Leitfähigkeit von höchstens 50 μS/cm, bevorzugt höchstens 30 μS/cm, besonders bevorzugt höchstens 20 μS/cm auf, insbesondere bevorzugt höchstens 10 μS/cm auf, jeweils gemessen bei 20°C.

Zum Ansäuern des eingesetzten Wassers können Brønstedt-Säuren oder Lewis-Säuren eingesetzt werden. Beispiele für Lewis-Säuren sind $BF_3$, $AlCl_3$, $TiCl_3$, $SnCl_4$, $SO_3$, $PCl_5$, $POCl_3$, $FeCl_3$ und dessen Hydrate und $ZnCl_2$.

Beispiele für Brønstedt-Säuren sind Salzsäure, Bromwasserstoffsäure, Schwefelsäure, Salpetersäure, salpetrige Säure, Chlorsulfonsäure, Phosphorsäuren, wie ortho-, meta- und Polyphosphorsäuren, Borsäure, selenige Säure, Salpetersäure, Carbonsäuren, wie Ameisensäure, Essigsäure, Propionsäure, Citronensäure und Oxalsäure, Halogenessigsäuren, wie Trichlorund Trifluoressigsäure, p-Toluolsulfonsäure, saure Ionenaustauscher, saure Zeolithe und säureaktivierte Bleicherde.

Bevorzugt sind Salzsäure, Bromwasserstoffsäure und Essigsäure.

Das Ansäuern des Wassers kann vor der Umsetzung zum Hydrolysat gleichzeitig mit der Umsetzung erfolgen oder sowohl vor der Umsetzung als auch gleichzeitig mit der Umsetzung erfolgen. In einer besonderen Ausführungsform wird das Wasser teilweise vor der Umsetzung zum Hydrolysat mit Salzsäure angesäuert und ein weiterer Teil Salzsäure wird durch die Trialkoxysilane der allgemeinen Formel (I) eingebracht.

Die Hydrolyse des Trialkoxysilans ist eine schwach exotherme Reaktion. Die Temperatur im ersten Schritt wird in einer bevorzugten Ausführungsform gegebenenfalls durch Heizen oder Kühlen vorzugsweise bei 0°C bis 60°C, bevorzugt bei 10°C bis 50°C, besonders bevorzugt bei 15°C bis 40°C, ganz besonders bevorzugt bei 15 bis 30 °C, insbesondere bei 15 - 25 °C gehalten, wobei die Temperaturschwankung nach Erreichen der Zieltemperatur vorzugsweise weniger als 10 °C, bevorzugt weniger als 5 °C beträgt. Die Dosierung des Trialkoxysilans kann beliebig vor oder nach Erreichen der Zieltemperatur begonnen werden.

In einer anderen Ausführungsform wird das Trialkoxysilan in einer Portion zudosiert. Dabei wird die Wärme nicht aktiv oder nur teilweise herausgekühlt. In dieser Ausführungsform findet eine exotherme Zunahme der Temperatur nach Zugabe des Trialkoxysilans statt. Die Temperatur der Umsetzung im ersten Schritt beträgt20 °C bis 80°C, vorzugsweise bis 60°C.

Vorzugsweise wird das Trialkoxysilan in 0,5 bis 5 h, besonders bevorzugt höchstens 2 h dosiert. Zwischen der schnellen Zugabe und der Dosierung gibt es einen fließenden Übergang an erfindungsgemäßen Ausführungsformen, d.h. es kann z.B. zügig in 15 min unter teilweiser Wärmeabführung bis maximal 40°C zugegeben werden, oder es kann z.B. über 2 h dosiert werden, dabei aber nur geringfügig gekühlt werden, wodurch man zunächst einen Temperaturanstieg auf 30°C zulässt und bei dieser Temperatur hält.
Besonders bevorzugt ist die Dosierung bei einer konstanten Temperatur.

Vorzugsweise werden im ersten Schritt auf 100 Gewichtsteile Wasser 5 bis 43 Gewichtsteile, vorzugsweise 11 bis 34 Gewichtsteile, insbesondere 13 bis 25 Gewichtsteile Trialkoxysilan eingesetzt.

Die Durchmischung im ersten Schritt kann durch einen statischen Mischer oder bevorzugt durch einen Rührer erfolgen.

Vorzugsweise wird nach Dosierung des Trialkoxysilans 5 min bis 5 h, besonders bevorzugt 10 min bis 3 h insbesondere 15 min bis 1,5 h nachgerührt. Die Nachrührzeit wird vorzugsweise so gewählt, dass die Summe der Zugabezeit des Silans und der Nachrührzeit 6 h nicht überschreiten.

Die Temperatur beim Nachrühren beträgt bei 0°C bis 60°C, bevorzugt bei 10°C bis 50°C, besonders bevorzugt bei 10°C bis 40°C, ganz besonders bevorzugt bei 10 bis 30 °C, insbesondere bei 15 bis 25 °C gehalten. Vorzugsweise ist die Differenz der Temperatur der Umsetzung im ersten Schritt und der Temperatur beim Nachrühren kleiner 20 °C, bevorzugt kleiner 10 °C, insbesondere kleiner 5 °C.

Vorzugsweise wird im zweiten Schritt eine Lösung von Alkalihydroxid in Wasser oder in einem Alkanol mit 1 bis 3 Kohlenstoffatomen eingesetzt. Bevorzugte Alkanole sind 1-Propanol, 2-Propanol, Ethanol und insbesondere Methanol. Eine Lösung von Alkalihydroxid in Wasser ist ebenfalls bevorzugt. Geeignet sind verdünnte oder konzentrierte Lösungen von Alkalihydroxid 0,001 bis 1100 g/l bei 20 °C, bevorzugt 0,01 bis 500 g/l, besonders bevorzugt 0,1 bis 500 g/l.

Beim Einsatz einer Lösung von Alkalihydroxid in einem Alkanol haften die Partikel weniger aneinander, zeigen einen niedrigeren Agglomerationsgrad und neigen weniger zum Verklumpen. Die Partikel zeigen ein in kosmetischen Anwendungen bevorzugtes trockneres Hautgefühl.

KOH ist als Alkalihydroxid bevorzugt.

Alternativ zu NaOH und KOH ist auch der Einsatz eines NaOH-oder KOH-Bildners möglich, der in zweiten Schritt mit dem im Hydrolysat vorhandenen Wasser sofort zu NaOH oder KOH reagiert. Beispiele dafür sind Natriumethanolat, Kaliummethanolat, NaH und KH. Bei dieser Ausführungsform ist der Einsatz von Natriumethanolat oder Kaliummethanolat in methanolischer Lösung bevorzugt.

Vorzugsweise wird so viel Lösung von Alkalihydroxid zugegeben, dass ein pH Wert von mindestens 6, vorzugsweise mindestens 6,5 und höchstens 10, vorzugsweise höchstens 9,5 erreicht wird, jeweils direkt nach Zugabe von Alkalihydroxid gemessen. Durch die Zugabe der Menge an Alkalihydroxid kann die Partikelgröße beeinflusst werden, wobei niedrige pH Werte größere Partikel ergeben. Der insbesondere bevorzugte pH Wert beträgt 7,5 bis 9.

Die Lösung von Alkalihydroxid wird vorzugsweise innerhalb von 10 Sekunden bis 10 Minuten, insbesondere innerhalb von 1 bis 3 Minuten zugegeben, vorzugsweise unter starkem und kurzem Rühren.

Die Temperatur der Zugabe von Alkalihydroxid im zweiten Schritt wird in einer bevorzugten Ausführungsform vorzugsweise bei 0°C bis 60°C, bevorzugt bei 10°C bis 50°C, besonders bevorzugt 10°C bis 40°C, ganz besonders bevorzugt bei 10°C bis 30°C, insbesondere bei 15°C bis 25°C gehalten. Vorzugsweise ist die Differenz der Temperatur beim Nachrühren und der Temperatur Zugabe von Alkalihydroxid kleiner 20 °C, bevorzugt kleiner 10 °C, insbesondere kleiner 5 °C.

Die Durchmischung im zweiten Schritt kann durch einen statischen Mischer oder bevorzugt durch einen Rührer erfolgen. Nach dem zweiten Schritt wird die Durchmischung vorzugsweise innerhalb von 10 Minuten, bevorzugt innerhalb von 5 Minuten beendet. Nach dem zweiten Schritt wird die Mischung vorzugsweise mindestens 1 h, bevorzugt mindestens 1,5 h, besonders bevorzugt mindestens 2,5 h nicht bewegt. Danach kann ein Rührer bei niedriger Drehzahl zugeschaltet werden, um ein Sedimentieren der Partikel zu verhindern. Dies ist optional und nicht notwendig, da sich die sedimentierten Polysilsesquioxanpartikel problemlos aufrühren lassen.

Nach dem zweiten Schritt wird die Temperatur der Mischung vorzugsweise mindestens 1 h, bevorzugt mindestens 1,5 h, besonders bevorzugt mindestens 2,5 h nicht mehr als 20 °C, bevorzugt nicht mehr als 10 °C verändert.

Wenn in der Anfangsphase im dritten Schritt, in welchem die Ausbildung der Partikel erfolgt, bewegt wird, treten vermehrt verformte, verwachsene oder agglomerierte Partikel auf.

In einer bevorzugten Ausführungsform wird die Mischung im dritten Schritt bis zur Isolierung der Polysilsesquioxanpartikel nicht bewegt.

Bevorzugt wird die Mischung im dritten Schritt mindestens 12 h, besonders bevorzugt mindestens 14 h, insbesondere mindestens 18 h aufbewahrt, bevor die Polysilsesquioxanpartikel isoliert werden. Auch Aufbewahrungszeiten bis 12 Wochen sind möglich.

Eine Trübung ist meist bereits nach 1 - 30 Minuten zu sehen.

Die Temperatur im dritten Schritt beträgt vorzugsweise 0°C bis 60°C, bevorzugt bei 10°C bis 50°C, besonders bevorzugt 10°C bis 40°C, ganz besonders bevorzugt 10°C bis 30°C, insbesondere 15°C bis 25°C. Bei niedrigen Temperaturen bilden sich größere Partikel, bei höheren Temperaturen bilden sich kleinere Partikel.

Bei einer Temperatur von 15°C bis 25°C besteht ein geringer oder kein Temperaturgradient der Reaktionsmischung zum Außenbereich, dadurch minimaler Wärmegradient zwischen Reaktorwandung und Reaktionslösung und dadurch minimierte thermische Konvektion während der Fällung der Partikel.

Das erfindungsgemäße Verfahren kann als Batchansatz, als Semi-Batch und oder als kontinuierlicher Prozess geführt werden.

Nach dem dritten Schritt werden die Partikel vorzugsweise isoliert, vorzugsweise durch Abfiltrieren oder Zentrifugieren. Nach dem Isolieren werden die Partikel vorzugsweise mit VE-Wasser oder Alkohol gewaschen und vorzugsweise getrocknet.

Die Trocknung erfolgt vorzugsweise bei 40 bis 250 °C, besonders bevorzugt bei 100 bis 240 °C, insbesondere bevorzugt bei 140 bis 220 °C. Die Trocknung kann unter Umgebungsdruck oder bei vermindertem Druck erfolgen. Während der Trocknung findet auch eine Kondensation freier Si-OH Gruppen statt, die laut Kinetikmessungen vorzugsweise ab 150 °C, besser ab 180 °C, ideal ab 200 °C abläuft. Partikel welche lange Zeit bei 100 °C getrocknet werden, sind zwar Trocken, weisen aber einen hohen Si-OH Gehalt auf. Bei 150 °C ist der Si-OH Gehalt deutlich reduziert, aber noch nicht vollständig entfernt, bei 200 °C werden Si-OH Gruppen nochmals signifikant reduziert. Durch einen reduzierten Si-OH Gehalt ergeben sich Vorteile im Verteilungsverhalten und der Fluidisierung der Partikel.

Die Partikel werden vorzugsweise 0,5 bis 100 h, besonders bevorzugt 0,5 bis 24 h insbesondere 1 bis 14 h getrocknet.

Eine besonders hohe Agglomerisationsfreiheit der Polysilsesquioxanpartikel kann durch eine anschließende Mahlung erreicht werden.

Die Polysilsesquioxanpartikel zeigen bei der Untersuchung im Elektronenmikroskop vorzugsweise eine kugelförmige Gestalt. Die sphärischen Polysilsesquioxanpartikel weisen vorzugsweise eine durchschnittliche Sphärizität y von mindestens 0,6, insbesondere mindestens 0,7 auf. Die sphärischen Polysilsesquioxanpartikel weisen vorzugsweise eine durchschnittliche Rundheit x von mindestens 0,6, insbesondere mindestens 0,7 auf. Die Rundheit x und Sphärizität y können nach DIN EN ISO 13503-2, Seite 37, Annex B.3, insbesondere Figur B.1 bestimmt werden.

Vorzugsweise werden alle Verfahrensschritte beim Druck der umgebenden Atmosphäre, also etwa 0,1 MPa (abs.) ausgeführt; sie können auch bei höheren oder niedrigeren Drücken durchgeführt werden. Bevorzugt sind Drücke von mindestens 0,08 MPa (abs.) und besonders bevorzugt mindestens 0,09 MPa (abs.), besonders bevorzugt höchstens 0,2 MPa (abs.), insbesondere höchstens 0,15 MPa (abs.).

Alle vorstehenden Symbole der vorstehenden Formeln weisen ihre Bedeutungen jeweils unabhängig voneinander auf. In allen Formeln ist das Siliciumatom vierwertig.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

**Beispiele**

Volumengewichtete Partikelgrößenverteilung dso

Die Bestimmung der volumengewichteten Partikelgrößenverteilung erfolgt nach ISO 13320 mittels statischer Laserstreuung mit dem Gerät Sympatec HELOS mit Trockendispergierer RODOS mit 2 bar Druckluft als Dispergiermedium. Der $d_{50}$ gibt dabei die mittlere Partikelgröße an.

Siebanalyse:

Die Siebanalyse erfolgte mittels Trockensiebung an einer Analysensiebmaschine vom Typ Retsch AS 200 basic bei 100 % Amplitude. Für die Analyse wurden vier Siebe nach DIN ISO 3310 folgender Maschenweite gestapelt: 200 $\mu$m, 100 $\mu$m, 40 $\mu$m, 20 $\mu$m, Boden. Es wurden jeweils 50 g Substanz auf dem obersten Sieb (200 $\mu$m) aufgebracht und für 10 Minuten gesiebt.

Bestimmung der Fluidisierung und Volumenzunahme:

In einen 50 ml PP-Zentrifugenröhrchen werden 6,0 g Polysilsesquioxanpartikel eingebracht, 30 Sekunden kräftig aufgeschüttelt und 1 h auf einer ebenen Fläche stehen gelassen. Danach wird, wenn nötig, durch leichtes Klopfen eine ebene Oberfläche erzeugt. Das Volumen(abgesetzt) der Probe wird abgelesen. Das Gebinde wird verschlossen und mindestens 30 Sekunden kräftig geschüttelt, bis sämtliches Material dispergiert ist. Das Zentrifugenröhrchen wird unmittelbar auf die Fläche zurückgestellt und sofort danach das Volumen(aufgeschüttelt) abgelesen. Aufschütteln und Ablesen wird insgesamt drei Mal wiederholt und aus den ermittelten Werten der Durchschnittswert Volumen (aufgeschüttelt, Durchschnittswert aus drei Versuchen) ermittelt. Die Volumenzunahme wird dann mit folgender Formel berechnet:

```
Volumenzunahme = Volumen (aufgeschüttelt, Durchschnittswert aus
drei Versuchen) x 100 / Volumen(abgesetzt)
```

Die mikroskopischen Untersuchungen wurden mit einem Rasterelektronemikroskop Zeiss SUPRA 55 VP durchgeführt. Die Proben wurden vor der Untersuchung mit einem Sputtercoater CCU-010 der Firma Safematic zur Verhinderung von Aufladungsphänomenen mit Gold besputtert.

Die sphärischen Polysilsesquioxanpartikel der Beispiele 1 bis 3 weisen eine durchschnittliche Sphärizität y von 0,8 und eine durchschnittliche Rundheit x von 0,85 auf nach DIN EN ISO 13503-2, Seite 37, Annex B.3, Figur B.1

Beispiel 1

1328 g vollentsalztes Wasser mit Leitfähigkeit 0,1 $\mu$S/cm wird in einem Glaskolben vorgelegt und auf 20 °C temperiert. Dabei wird mit 300 Upm gerührt. Der pH wird durch Zugabe von 0,1 molarer Salzsäure auf einen Wert von 4,40 eingestellt. 291,6 g Methyltrimethoxysilan werden über 1 h zudosiert, dabei wird die Temperatur auf 20 °C gehalten. Nach Ende der Dosierung wird 1 h bei 20 °C gerührt. Es werden 65,49 g 0,1 molare wäßrige KOH-Lösung innerhalb 1 min bei 20 °C zugeben und insgesamt 3 min homogen vermischt. Dann wird der Rührer abgestellt. Nach 21 h werden die ausgefallenen Partikel abfiltriert, mit VE-Wasser gewaschen und bei 150 °C 18 h im Trockenschrank getrocknet.

Beispiel 2

Die Durchführung erfolgt nach Verfahren von Beispiel 1, jedoch werden zur Fällung 65,84 g 0,1 molare wässrige NaOH-Lösung zugegeben.

Beispiel 3

Die Durchführung erfolgt nach Verfahren von Beispiel 1, jedoch werden zur Fällung 65,49 g 0,1 molare methanolische KOH -Lösung zugegeben.

Vergleichsbeispiel 1, (nicht erfindungsgemäß)

Die Durchführung erfolgt nach Verfahren von Beispiel 1. Zur Fällung wird die gleiche Base verwendet, jedoch werden die ausgefallenen Partikel bereits nach 4 h Haltezeit nach Basenzugabe abfiltriert, mit VE-Wasser gewaschen und bei 150 °C 18 h im Trockenschrank getrocknet.

Vergleichsbeispiel 2, (nicht erfindungsgemäß)

Die Durchführung erfolgt nach Verfahren von Beispiel 2. Zur Fällung wird die gleiche Base verwendet, jedoch werden die ausgefallenen Partikel bereits nach 4 h Haltezeit nach Basenzugabe abfiltriert, mit VE-Wasser gewaschen und bei 150 °C 18 h im Trockenschrank getrocknet.

Vergleichsbeispiel 3, (nicht erfindungsgemäß)

Die Durchführung erfolgt nach Verfahren von Beispiel 1, jedoch werden zur Fällung 7,96 g 1 molare wässrige Ammoniaklösung zugegeben.

Vergleichsbeispiel 4, (nicht erfindungsgemäß)

Die Durchführung erfolgt nach Verfahren von Beispiel 3. Zur Fällung wird die gleiche Base verwendet, jedoch werden die ausgefallenen Partikel bereits nach 4 h Haltezeit nach Basenzugabe abfiltriert, mit VE-Wasser gewaschen und bei 150 °C 18 h im Trockenschrank getrocknet.

Vergleichsbeispiel 5, (nicht erfindungsgemäß)

Die Durchführung erfolgt nach Verfahren von Beispiel 1, jedoch werden zur Fällung 71,73 g 0,1 molare wässrige Diethylamin-Lösung zugegeben.

Vergleichsbeispiel 6, (nicht erfindungsgemäß)

Die Durchführung erfolgt nach Verfahren von Beispiel 1, jedoch werden zur Fällung 243,99 g 0,1 molare wässrige Calciumhydroxid-Lösung zugegeben.

Vergleichsbeispiel 7, (nicht erfindungsgemäß)

Die nicht erfindungsgemäßen Partikel hergestellt nach Vergleichsbeispiel V3 wurden mit einer Fließbett-Gegenstrahlmühle vom Typ Alpine 100AFG bei 7 bar Druck und einer Sichterdrehzahl von 12000 U/min dispergiert.

Tabelle 1

| Beispiel | | 1 | 2 | 3 | V1* | V2* | V3* | V4* | V5* | V6* | V7* |
|---|---|---|---|---|---|---|---|---|---|---|---|
| Fällungsbase | | KOH | NaOH | KOH (MeOH) | KOH | NaOH | Ammoniak | Ammoniak | Diethylamin | Ca (OH) 2 | Ammoniak |
| Fällungszeit** | Std. | 21 | 21 | 21 | 4 | 4 | 21 | 4 | 21 | 21 | 21 |
| Mahlung | | nein | nein | nein | nein | nein | nein | nein | nein | nein | ja |
| Siebfraktion | <20 $\mu$m | 68 | 59 | 67 | 0 | 0 | 1 | 0 | 4 | 25 | 35 |
| Siebfraktion | 20 - 40 $\mu$m | 10 | 14 | 17 | 0 | 0 | 16 | 0 | 55 | 19 | 55 |
| Siebfraktion | 40 - 100 $\mu$m | 10 | 14 | 9 | 8 | 5 | 41 | 7 | 16 | 18 | 5 |
| Siebfraktion | 100 - 200 $\mu$m | 7 | 6 | 5 | 33 | 32 | 27 | 31 | 13 | 20 | 3 |
| Siebfraktion | >200 $\mu$m | 5 | 7 | 3 | 58 | 62 | 15 | 62 | 12 | 18 | 2 |
| Summe Anteil <40 $\mu$m | | 78 | 73 | 84 | 0 | 0 | 17 | 0 | 59 | 44 | 90 |
| Summe Anteil >100 $\mu$m | | 12 | 13 | 8 | 91 | 94 | 68 | 93 | 25 | 37 | 5 |
| Mittlere Partikelgröße $d_{50}$ | $\mu$m | 4,10 | 4,05 | 4,45 | 3,94 | 4,04 | 4,22 | 5,29 | 5,30 | 4,14 | 4,22 |
| Fluidisierung | | ja | ja | ja | nein | nein | ja | nein | ja | ja | ja |
| Volumenzunahme | % | 170 | n.B. | 210 | 0 | 0 | 130 | 0 | n.B. | n.B. | n.B. |

n.B. nicht bestimmt
* nicht erfindungsgemäß
** Haltezeit in Stunden nach Basenzugabe

EP 3 377 558 B1

Beispiel 4: Bewertung der sensorischen Eigenschaften

Die Bewertung der sensorischen Eigenschaften der erfindungsgemäßen sphärischen Polysilsesquioxanpartikel aus den Beispielen 1 und 3 und der nicht- erfindungsgemäßen Polysilsesquioxanpartikel aus dem Vergleichsbeispiel V3 erfolgte durch eine geschulte Gruppe von fünf Probanden. Bewertet wurden die Leichtigkeit der Verteilbarkeit der Partikel und die Neigung beim Verteilen zu Verklumpen oder zu Agglomerieren. Bewertet wurde mit einer Skala von 0 bis 2, wobei 2 die beste Verteilbarkeit und niedrigste Agglomerationsneigung bedeutet.

Tabelle 2

| Beispiel | Verteilbarkeit | Agglomerationsneigung |
|---|---|---|
| Beispiel 1 | 1 | 1 |
| Beispiel 3 | 2 | 2 |
| Beispiel V3 | 0 | 0 |

Nach dem Verteilen wurden die sensorischen Eigenschaften des jeweiligen Rückstands bewertet.
Die Rückstände der erfindungsgemäßen Polysilsesquioxanpartikel aus den Beispielen 1 und 3 wurden jeweils als bevorzugt seidigsamtig bewertet, wobei Beispiel 3 ein im Vergleich stärker ausgeprägtes trockenes Hautgefühl aufweist, welches für kosmetische Anwendungen besonders bevorzugt ist. Der Rückstand der nicht-erfindungsgemäßen Polysilsesquioxanpartikel aus dem Vergleichsbeispiel V3 wurde als ungeeignet wachsig-kreidig bewertet.

**Patentansprüche**

1.   Verfahren zur Herstellung sphärischer Polysilsesquioxanpartikel, bei dem in einem ersten Schritt Trialkoxysilan der allgemeinen Formel (I)

$$RSi(OR^1)_3 \qquad (I),$$

in der

R einen Kohlenwasserstoffrest mit 1 bis 16 Kohlenstoffatomen dessen Kohlenstoffkette durch nicht benachbarte Gruppen -O-unterbrochen sein kann,
$R^1$ einen $C_1$- bis $C_4$-Alkylrest bedeuten,
mit angesäuertem Wasser mit einem pH-Wert von höchstens 6 unter Durchmischung zu einem Hydrolysat umgesetzt wird,
in einem zweiten Schritt das Hydrolysat mit einer Lösung von Alkalihydroxid, das ausgewählt wird aus NaOH und KOH oder einem Gemisch davon vermischt wird
und in einem dritten Schritt die Mischung mindestens 8 h aufbewahrt wird bevor die Polysilsesquioxanpartikel isoliert werden.

2.   Verfahren nach Anspruch 1, bei dem R Ethylrest oder Methylrest bedeutet.

3.   Verfahren nach einem oder mehreren der voranstehenden Ansprüche, bei dem $R^1$ einen Ethylrest oder Methylrest bedeutet.

4.   Verfahren nach einem oder mehreren der voranstehenden Ansprüche, bei dem die Umsetzung zum Hydrolysat bei einem pH-Wert von 4,5 bis 2 erfolgt.

5.   Verfahren nach einem oder mehreren der voranstehenden Ansprüche, bei dem die Temperatur der Umsetzung im ersten Schritt 0°C bis 60°C beträgt.

6.   Verfahren nach einem oder mehreren der voranstehenden Ansprüche, bei dem im ersten Schritt auf 100 Gewichtsteile Wasser 5 bis 43 Gewichtsteile Trialkoxysilan eingesetzt werden.

7.   Verfahren nach einem oder mehreren der voranstehenden Ansprüche, bei dem im zweiten Schritt eine Lösung von Alkalihydroxid in Wasser oder in einem Alkanol mit 1 bis 3 Kohlenstoffatomen eingesetzt wird.

8.  Verfahren nach einem oder mehreren der voranstehenden Ansprüche, bei dem so viel Lösung von Alkalihydroxid zugegeben wird, dass ein pH Wert von 6,5 bis 9,5 erreicht wird, jeweils direkt nach Zugabe von Alkalihydroxid gemessen.

9.  Verfahren nach einem oder mehreren der voranstehenden Ansprüche, bei dem die Temperatur der Zugabe von Alkalihydroxid im zweiten Schritt 10°C bis 40°C beträgt.

10. Verfahren nach einem oder mehreren der voranstehenden Ansprüche, bei dem im dritten Schritt die Mischung mindestens 14 h aufbewahrt wird bevor die Polysilsesquioxanpartikel isoliert werden.

11. Verfahren nach einem oder mehreren der voranstehenden Ansprüche, bei dem die Partikel nach dem dritten Schritt durch Abfiltrieren oder Zentrifugieren isoliert werden.

**Claims**

1.  Process for producing spherical polysilsesquioxane particles in which in a first step trialkoxysilane of general formula, (I)

    $$RSi(OR^1)_3 \qquad (I),$$

    in which

    **R** represents a hydrocarbon radical having 1 to 16 carbon atoms whose carbon chain may be interrupted by nonadjacent -O- groups,
    **R$^1$** represents a $C_1$- to $C_4$-alkyl radical,
    is reacted with acidified water having a pH of not more than 6 with stirring to afford a hydrolysate,
    in a second step the hydrolysate is mixed with a solution of alkali metal hydroxide selected from NaOH and KOH or a mixture thereof
    and in a third step the mixture is stored for at least 8 h before the polysilsesquioxane particles are isolated.

2.  Process according to claim 1, in which **R** represents ethyl radical or methyl radical.

3.  Process according to claim 1 one or more of the preceding claims, in which **R$^1$** represents an ethyl radical or methyl radical.

4.  Process according to one or more of the preceding claims, in which the reaction to afford the hydrolysate is effected at a pH of 4.5 to 2.

5.  Process according to one or more of the preceding claims in which the temperature of the reaction in the first step is 0°C to 60°C.

6.  Process according to one or more of the preceding claims in which in the first step 5 to 43 parts by weight of trialkoxysilane are added per 100 parts by weight of water.

7.  Process according to one or more of the preceding claims in which in the second step a solution of alkali metal hydroxide in water or in an alkanol having 1 to 3 carbon atoms is employed.

8.  Process according to one or more of the preceding claims in which sufficient solution of alkali metal hydroxide is added to ensure that a pH of 6.5 to 9.5 is achieved in each case immediately after addition of alkali metal hydroxide.

9.  Process according to one or more of the preceding claims in which the temperature of the addition of alkali metal hydroxide in the second step is 10°C to 40°C.

10. Process according to one or more of the preceding claims in which in the third step the mixture is stored for at least 14 h before the polysilsesquioxane particles are isolated.

**11.** Process according to one or more of the preceding claims in which after the third step the particles are isolated by filtration or centrifugation.

**Revendications**

**1.** Procédé pour la préparation de particules sphériques de polysilsesquioxane, dans lequel, dans une première étape, du trialcoxysilane de formule générale (I)

$$RSi(OR^1)_3 \qquad (I),$$

dans laquelle

R signifie un radical hydrocarboné comprenant 1 à 16 atomes de carbone dont la chaîne carbonée peut être interrompue par des groupes -O- non adjacents,
$R^1$ signifie un radical $C_1$-$C_4$-alkyle,
est transformé avec de l'eau acidifiée présentant un pH d'au plus 6 avec mélange en un hydrolysat, dans une deuxième étape, l'hydrolysat est mélangé avec une solution d'hydroxyde de métal alcalin qui est choisi parmi NaOH et KOH ou un mélange de ceux-ci et, dans une troisième étape, le mélange est conservé pendant au moins 8 h avant l'isolement des particules de polysilsesquioxane.

**2.** Procédé selon la revendication 1, dans lequel R signifie un radical éthyle ou un radical méthyle.

**3.** Procédé selon l'une ou plusieurs des revendications précédentes, dans lequel $R^1$ représente un radical éthyle ou un radical méthyle.

**4.** Procédé selon l'une ou plusieurs des revendications précédentes, dans lequel la transformation en hydrolysat a lieu à un pH de 4,5 à 2.

**5.** Procédé selon l'une ou plusieurs des revendications précédentes, dans lequel la température de la transformation dans la première étape est de 0°C à 60°C.

**6.** Procédé selon l'une ou plusieurs des revendications précédentes, dans lequel on utilise, dans la première étape, pour 100 parties en poids d'eau, 5 à 43 parties en poids de trialcoxysilane.

**7.** Procédé selon l'une ou plusieurs des revendications précédentes, dans lequel on utilise, dans la deuxième étape, une solution d'hydroxyde de métal alcalin dans de l'eau ou dans un alcanol comprenant 1 à 3 atomes de carbone.

**8.** Procédé selon l'une ou plusieurs des revendications précédentes, dans lequel on ajoute une quantité de solution d'hydroxyde de métal alcalin telle qu'on atteint un pH de 6,5 à 9,5, à chaque fois directement après l'addition d'hydroxyde de métal alcalin.

**9.** Procédé selon l'une ou plusieurs des revendications précédentes, dans lequel la température de l'addition d'hydroxyde de métal alcalin dans la deuxième étape est de 10°C à 40°C.

**10.** Procédé selon l'une ou plusieurs des revendications précédentes, dans lequel, dans la troisième étape, le mélange est conservé pendant au moins 14 h avant l'isolement des particules de polysilsesquioxane.

**11.** Procédé selon l'une ou plusieurs des revendications précédentes, dans lequel les particules sont isolées après la troisième étape par séparation par filtration ou par centrifugation.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 3970449 B
- JP H1045914 A
- JP 2003335860 A
- JP H06248081 A
- JP H0488023 A